# EUROPEAN PATENT SPECIFICATION

(11) **EP 4 034 195 B1**
(45) Date of publication and mention of the grant of the patent: **19.11.2025**
(21) Application number: 20790077.0
(22) Date of filing: 24.09.2020
(51) Int. Cl.: A61M 5/145, A61M 5/142, A61M 5/168, G16H 20/17, G16H 40/63, A61M 5/172

(54) **MULTI-RATE DRUG DELIVERY DEVICE AND METHOD OF CONTROLLING THE DEVICE**
MEHRFACH-RATEN ARZNEIMITTELABGABEVORRICHTUNG UND VERFAHREN ZUM STEUERN DER VORRICHTUNG
DISPOSITIF D'ADMINISTRATION DE MÉDICAMENTS À DÉBIT MULTIPLE ET MÉTHODE DE CONTRÔLE DE CE DISPOSITIF

(30) Priority: 27.09.2019 GB 201913995
(43) Date of publication of application: 03.08.2022
(73) Proprietor: West Pharma. Services IL, Ltd., 4366411 Ra'anana (IL)
(72) Inventor: VATELMACHER, Michael, 4366411 Ra'anana (IL); GERBI, Eliran, 4366411 Ra'anana (IL); ISAKOV, Igor, 4366411 Ra'anana (IL)
(74) Representative: Finlayson, Scott Henry
(86) International application number: PCT/IL2020/051045
(87) International publication number: WO 2021/059277

(56) References cited:
- US-A1- 2011 137 239
- US-A1- 2012 123 229
- US-A1- 2013 281 965
- US-A1- 2014 094 770
- US-A1- 2018 085 520

## Description

### Technical Field

The present invention relates to systems and methods for controlling a drive system in a drug delivery device. More particularly, the invention relates to systems and methods for controlling a drive system configured to deliver a first volume of medicament at a first rate and a second volume of medicament at a second rate, and drug delivery devices comprising the drive system.

### Background

The management and treatment of many medical conditions, in particular chronic conditions, may require delivery of a medicament via injection. Often, medicament may be delivered outside of a traditional healthcare setting, and in many cases without a healthcare professional in attendance throughout the injection process. Limitations impacting the management and control of medicament delivery outside of traditional healthcare settings include patient compliance with prescribed dosage regimens and the limitations placed on the delivery profile of a medicament by the traditional injection apparatuses used (e.g. total volume, delivery time, etc). Poor patient compliance or restricted delivery profiles may provide an impediment to achieving a particular pharmacokinetic (or pharmacodynamic) profile for a particular medicament.

Drug delivery devices that allow for controlled delivery of medicament over an extended period (for example several minutes, hours or days) have been developed.

US2018/085520 describes an infusion mode for an infusion pump that is configured for the scheduling of infusions without requiring the clinician to hand-calculate and manually update the infusion pump, should a stoppage occur. Consideration of a flush time (the duration of time for the flush) and flush rate (the rate for the flush), as part of the infusion is further included in the infusion scheduling.

US2014/094770 describes a closed-loop feedback control system to ensure accurate drug delivery. This control system may, for example, utilize a flow sensor to measure the volume of delivery and an intelligent control algorithm to anticipate and compensate for overdoses and underdoses. Feedback control systems in accordance herewith can be applied to any piston- or plunger-driven pump system utilizing sensors that measure flow directly or indirectly. In some embodiments, adjustments are made during a "priming" stage when liquid is pumped through the internal fluid path but does not exit the pump.

US2012/123229 describes a medication delivery monitoring which includes a user interface configured to receive input information, and a sensor configured to measure a plurality of fluid state parameters of a fluid delivery channel through which the medication is delivered by a vascular access device (VAD) to an infusion site region of the patient. The device also includes a processor configured to determine a state of the infusion site region based on the plurality of measured fluid state parameters and the input information, and an output device configured to provide a communication regarding the state of the infusion site region.

### Summary of invention

According to a first aspect of the invention, there is provided a drug delivery device comprising a housing configured to receive a container containing a volume of medicament; an actuator configured to initiate a drug delivery procedure; and a drive system comprising: an axial drive component configured to drive advancement of a stopper sealing the container; a variable rate drive configured to drive advancement of the axial drive component; a controller configured to drive the variable rate drive to deliver a first pre-defined volume V1 of medicament from the container at a first set rate R1, and a second predefined volume V2 of medicament from the container at a second set rate R2, and wherein the controller comprises a processor configured to (a) drive the variable rate drive at a first speed during a first phase P1, to deliver the first volume V1 of medicament at the first rate R1; (b) identify an end point for the first phase P1; and (c) drive the variable rate drive at a second speed during a second phase P2 to deliver the second volume V2 of medicament at the second rate R2, wherein the second phase begins at or after the identified endpoint of the first phase. The first and second volumes V1, V2 of medicament are delivered from the same container. The first and second volumes V1, V2 may be different, or they may be the same. The first and second rates are different.

By providing a drug delivery device configured to supply a first predefined volume of medicament at a first rate, and a second predefined volume of medicament at a second rate once completion of the first dose is identified, the present invention may allow precise control of the dosage profile for a drug that requires (or has improved efficacy) when delivered according to a multi-rate delivery profile.

The end-point for the first phase P1 is identified as the delivery of the first pre-determined volume. This can be determined based on a measured condition of the drive system, for example the number of turns of a motor.

Optionally, the controller may be further configured to identify a start point for delivery of medicament from the container, wherein the start point is identified by identifying completion of a predefined preparatory event, and wherein the first phase P1 begins at the identified start point.

By providing a drug delivery device in which a start point of injection is identified based on a predetermined parameter, the rate of drug delivery for a first volume of medicament can be more precisely controlled during a first phase. Moreover, by identifying an end point of the first phase (after which the first volume has been delivered), the rate of delivery of a second volume of medicament can be controlled at a second rate during a second phase. Such a device can allow for control of drug delivery of first and second volumes of medicament (from the same container) at different rates.

In any of the embodiments and aspects described herein, the variable rate drive may comprise a motor, e.g. an electric motor, configured to drive (directly or indirectly) the axial drive component. Optionally, the drive system comprises a telescopic screw assembly.

The motor may be DC motor, and in some embodiments, a pulse width modulation (PWM) driven motor. Optionally, the motor can be a constant power motor. Other motor control systems are also possible.

The predetermined preparatory event can be one (or more) of: completion of a predetermined distance travelled by the axial drive component; completion of a predetermined number of motor rotations; and/or completion of a pre-determined time-period.

The predetermined preparatory event can be determined based on a number of rotations of a motor that drives the variable rate drive. The number of rotations of the motor can be counted based on the pulses generated by an encoder associated with the motor, for example an optical encoder. A similar measure can be used to determine the end point of the first phase (when the first volume of medicament has been delivered). By directly counting the motor rotations to identify the start point for injection, and to identify the end of the first phase, the control system according to the present invention allows for precise control of the volume of drug delivered, and the rate at which the drug is delivered, since the start and end points of each phase of a multi-rate drug delivery profile is known. This can allow enhanced compliance with prescribed dosage regimes that may not be possible in other systems.

The drug delivery device may therefore comprise an encoder, for example an optical encoder, configured to monitor the speed of the motor and provide feedback to the controller. The encoder can be configured such that 6 or more pulses, and more preferably 8 or more pulses represents one rotation of the motor.

The controller can be configured to adjust the power supplied to the motor in response to the measured speed of the motor.

In some embodiments, the controller can be configured to control the variable rate drive to run at: a preparatory speed during a preparatory phase (P0) until completion of the predetermined preparation event; a first speed (S1) during the first phase (P1); and a second speed (S2) during the second phase (P2). During the preparatory phase (before identification of the start of drug delivery), the motor drives the axial drive component towards the stopper and closes the gap between the axial drive component and the stopper. The motion of the axial drive component during this period may therefore be considered idle, and the motor may be configured to run at a higher speed than during drug delivery. Accordingly, the preparatory speed may be greater than S1 and S2.

The controller may be configured to drive the variable rate drive during a medicament delivery period T_{D}, defined as a time taken to complete the first phase (T_{P1}) and a time taken to complete the second phase (T_{P2}). T_{D} may be several minutes or several hours, or in some embodiments, several days. For example, T_{D} may be at least 2.5 minutes, at least 5 minutes, at least 10 minutes, at least 30 minutes, at least 60 minutes, at least 120 mins, at least 240 minutes, or at least 300 minutes.

In a second aspect of the invention, there is provided a drive system for an injection device comprising: an axial drive component configured to drive advancement of a stopper sealing the container; a variable rate drive configured to drive advancement of the axial drive component; a controller configured to drive the variable rate drive to deliver a first pre-defined volume V1 of medicament from the container at a first rate R1, and a second predefined volume V2 of medicament from the container at a second rate R2, and wherein the controller comprises a processor configured to: (a) drive the variable rate drive at a first speed during a first phase P1, to deliver the first volume V1 of medicament at the first rate R1; (b) identify an end point for the first phase P1; and (c) drive the variable rate drive at a second speed during a second phase P2 to deliver the second volume V2 of medicament at the second rate R2, wherein the second phase begins after the identified endpoint of the first phase.

Optionally, the controller may be further configured to identify a start point for delivery of medicament from the container, wherein the start point is identified by identifying completion of a predefined preparatory event, and wherein the first phase P1 begins at the identified start point.

The variable rate drive may comprise a motor configured to drive the axial drive component. Optionally, the drive system comprises a telescopic screw assembly.

The motor may be a pulse width modulation (PWM) driven motor. Optionally, the motor can be a constant power motor. Other motor control systems are also possible.

The predetermined preparatory event can be one (or more) of: completion of a predetermined distance travelled by the axial drive component; completion of a predetermined number of motor rotations; and/or completion of a pre-determined time-period.

The predetermined preparatory event can be determined based on a number of rotations of a motor that drives the variable rate drive. The number of rotations of the motor can be counted based on the pulses generated by an encoder associated with the motor, for example an optical encoder. A similar measure can be used to determine the end point of the first phase (when the first volume of medicament has been delivered).

The drug delivery device may therefore comprise an optical encoder to monitor the speed of the motor and provide feedback to the controller.

The controller can be configured to adjust the power supplied to the motor in response to the measured speed of the motor.

In some embodiments, the controller can be configured to control the variable rate drive to run at: a preparatory speed during a preparatory phase (P0) until completion of the predetermined preparation event; a first speed (S1) during the first phase (P1); and a second speed (S2) during the second phase (P2). During the preparatory phase (before identification of the start of drug delivery), the motor drives the axial drive component towards the stopper and closes the gap between the axial drive component and the stopper. The motion of the axial drive component during this idle period may therefore be considered idle, and the motor may be configured to run at a higher speed that during drug delivery. Accordingly, the preparatory speed may be greater than S1 and S2.

The controller may be configured to drive the variable rate drive during a medicament delivery period T_{D}, defined as a time taken to complete the first phase (T_{P1}) and a time taken to complete the second phase (T_{P2}). T_{D} may be several minutes or several hours, or in some embodiments, several days. For example, T_{D} may be at least 2.5 minutes, at least 5 minutes, at least 10 minutes, at least 30 minutes, at least 60 minutes, at least 120 mins, at least 240 minutes, or at least 300 minutes.

In a third aspect of the invention, there is provided a method for controlling a drive system for a drug delivery device, wherein the drive system comprises an axial drive component configured to drive advancement of a stopper sealing a container, a variable rate drive configured to drive advancement of the axial drive component, and a controller configured to drive the variable rate drive, the method comprising: driving the variable rate drive at a first speed during a first phase (P1) from an identified start point to advance the axial drive component a first axial distance at a first rate; identify an end point for the first phase P1; and driving the variable rate drive at a second speed during a second phase (P2) to drive the axial drive component a second axial distance at a second rate, wherein the first speed is different to the second speed.

Optionally, the method may further comprise: identifying engagement of the axial drive component with a stopper by identifying completion of a predefined preparatory event.

The predefined preparatory event may be completion of a predetermined distance travelled by the axial drive component. In some embodiments, the predefined preparatory event can be completion of a predetermined number of motor rotations.

Optionally, the drive system further comprises a measurement device for measuring a speed of rotation of the motor and the controller can be configured to vary the power supplied to the motor to maintain the measured motor speed at the first speed during the first phase and the second speed during the second phase.

The method may further comprise controlling the variable rate drive to run at: a first speed until completion of the predetermined preparation event; a second speed during the first phase; and a third speed during the second phase. In some embodiments, S1 may be greater than S2 and S3. The controller may control the variable rate drive during a medicament delivery period T_{D}, defined as a time taken to complete the first phase (T_{P1}) and a time taken to complete the second phase (T_{P2}). T_{D} may be several minutes or several hours, or in some embodiments, several days. For example, T_{D} may be at least 2.5 minutes, at least 5 minutes, at least 10 minutes, at least 30 minutes, at least 60 minutes, at least 120 mins, at least 240 minutes, or at least 300 minutes.

In a fourth aspect of the invention, there is provided a method of delivering a dose of medicament using drug delivery device. According to the fourth aspect, the method comprises providing a drug delivery device comprising a container holding a dose of medicament to be delivered to a patient. The method further comprises providing a drive system comprising: an axial drive component configured to drive advancement of a stopper sealing the container; a variable rate drive configured to drive advancement of the axial drive component; a controller configured to drive the variable rate drive. The controller delivers a pre-defined volume V1 of medicament from the container at a first rate R1, and a second predefined volume V2 of medicament from the container at a second rate R2. The controller further (a) drives the variable rate drive at a first speed during a first phase P1, to deliver the first volume V1 of medicament at the first rate R1, wherein the first phase P1 begins at the identified start point; (b) identifies an end point for the first phase P1; and (c) drives the variable rate drive at a second speed during a second phase P2 to deliver the second volume V2 of medicament at the second rate R2, wherein the second phase begins after the identified endpoint of the first phase.

Optionally, the controller may be further configured to identify a start point for delivery of medicament from the container, wherein the start point is identified by identifying completion of a predefined preparatory event, and wherein the first phase P1 begins at the identified start point.

The variable rate drive may comprise a motor configured to drive the axial drive component. Optionally, the drive system comprises a telescopic screw assembly.

The motor may be a pulse width modulation (PWM) driven motor. Optionally, the motor can be a constant power motor. Other motor control systems are also possible.

The predetermined preparatory event can be one (or more) of: completion of a predetermined distance travelled by the axial drive component; completion of a predetermined number of motor rotations; and/or completion of a pre-determined time-period.

The predetermined preparatory event can be determined based on a number of rotations of a motor that drives the variable rate drive. The number of rotations of the motor can be counted based on the pulses generated by an encoder associated with the motor, for example an optical encoder. A similar measure can be used to determine the end point of the first phase (when the first volume of medicament has been delivered).

The drug delivery device may therefore comprise an optical encoder to monitor the speed of the motor and provide feedback to the controller.

The controller may adjust the power supplied to the motor in response to the measured speed of the motor.

In some embodiments, the controller can control the variable rate drive to run at: a preparatory speed during a preparatory phase (P0) until completion of the predetermined preparation event; a first speed (S1) during the first phase (P1); and a second speed (S2) during the second phase (P2). During the preparatory phase (before identification of the start of drug delivery), the motor drives the axial drive component towards the stopper and closes the gap between the axial drive component and the stopper. The motion of the axial drive component during this idle period may therefore be considered idle, and the motor may be configured to run at a higher speed that during drug delivery. Accordingly, the preparatory speed may be greater than S1 and S2.

The controller may drive the variable rate drive during a medicament delivery period T_{D}, defined as a time taken to complete the first phase (T_{P1}) and a time taken to complete the second phase (T_{P2}). T_{D} may be several minutes or several hours, or in some embodiments, several days. For example, T_{D} may be at least 2.5 minutes, at least 5 minutes, at least 10 minutes, at least 30 minutes, at least 60 minutes, at least 120 mins, at least 240 minutes, or at least 300 minutes.

In some embodiments, the controller of the drug delivery device is further configured to drive the variable rate drive to deliver a third predefined volume V3 of medicament from the container at a third rate R3. The controller may comprise a processor and be configured to identify an end point for the second phase P2, and drive the variable rate drive at a third speed during a third phase P3, to deliver the third volume V3 of medicament at the third rate R3. In such an embodiment, the total volume of medicament to be delivered may be the total of the first V1, second V2, and third V3 volumes (i.e. V1+V2+V3).

In some embodiments, the controller is configured to drive the variable rate drive to deliver a tuple predefined volume Vn of medicament from the container at a tuple rate Rn. The controller comprises a processor and is configured to identify an end point for the phase Pn-1 before a tuple phase Pn; and drive the variable rate drive at a tuple speed during the tuple phase Pn, to deliver the tuple volume Vn of medicament at the tuple rate Rn. The tuple volume may be any positive non-integer list of consecutive volumes up to n. For example, where a total of three volumes is the tuple, then V1+V2+V3 will be the total volume; where a total of five volumes is the tuple, then V1+V2+V3+V4+V5 will be the total volume; and where a total of n volumes is the tuple, then V1+V2+...+Vn will be the total volume.

### Brief description of the drawings

Illustrative, non-limiting embodiments of the invention will now be described with reference to the following drawings, in which:
Fig. 1 shows an exploded view of a drug delivery device comprising a drive system according to an embodiment of the invention;
Fig. 2A shows a schematic view of a drive system in accordance with an embodiment of the invention, coupled with a medicament container;
Fig. 2B shows a partially exploded view of the drive system shown in Fig. 2A;
Fig. 3 shows the speed of a drive according to an embodiment throughout an exemplary infusion cycle;
Fig. 4 shows the volume of medicament delivered by the drive speed profile shown in Fig. 3;
Fig. 5 shows the steps of a method of controlling a drive system according to an embodiment of the present invention;
Fig. 6 shows the speed of a drive according to an embodiment throughout an exemplary infusion cycle;
Fig. 7 shows the volume of medicament delivered by the drive speed profile shown in Fig. 6;
Fig. 8 shows the speed of a drive according to an embodiment throughout an exemplary infusion cycle;
Fig. 9 shows the volume of medicament delivered by the drive speed profile shown in Fig. 8;
Fig. 10 shows a schematic view of an encoder; and
Fig. 11 shows a schematic view of a drive system in accordance with an embodiment of the invention, coupled with a medicament container. Fig. 11 also shows a needle.

### Detailed description of the invention

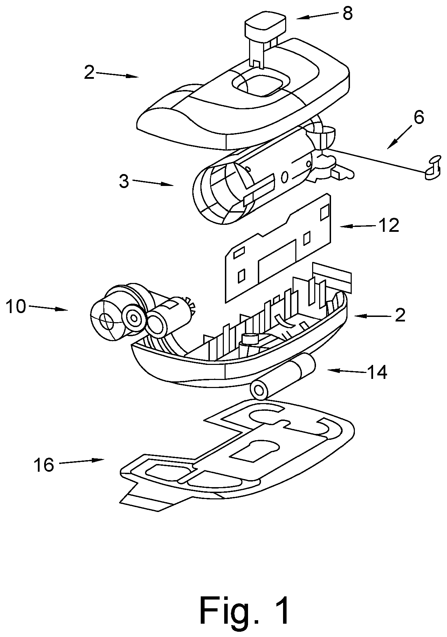

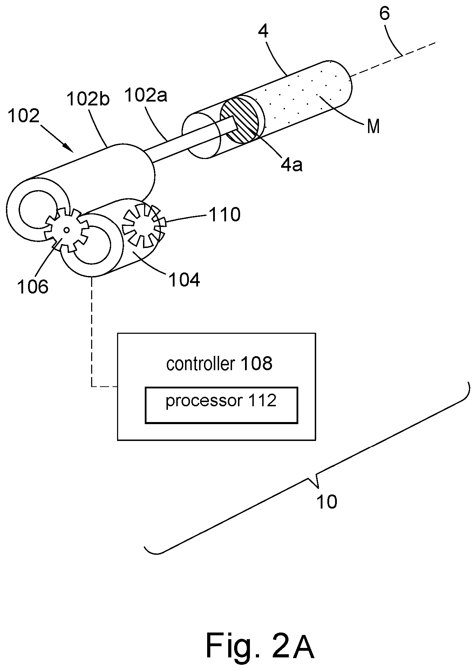

The present disclosure is directed generally to systems and methods for controlling a drive system in a drug delivery device. The disclosure also includes a drug delivery device comprising a drive system according to the disclosure. As shown in Figure 1, an exemplary drug delivery device in which the present invention may be implemented comprises a housing 2 containing a compartment 3 configured to receive a medicament container 4 (see, Fig. 2A), a fluid line 6 in fluid communication with a needle (not shown), an actuator 8 to initiate a drug delivery process, and a drive system 10. The housing 2 also contains a printed circuit board assembly (PCBA) 12 comprising a microcontroller for controlling the components of the drive system 10, and a battery 14 to power the device. The device can be configured as a wearable device and may include an adhesive layer 16 to secure the device to the user's skin. It will be appreciated that the housing 2 can also be secured to the patient's skin by other means.

The actuator 8 is configured to initiate a drug delivery procedure. In the embodiment shown in Figure 1, the actuator 8 is illustrated as a button on an exterior surface of the housing 2. However, it will be appreciated that the actuator 8 may be an internal component activated by an external button provided on the housing, or by a remotely or wirelessly connected device.

In some embodiments, the device can be designed for disposal after a single use, or it may be configured such that the container 4 and/or the needle can be removed and replaced, and the drive system reset.

The drive system 10 and the container 4 will now be described in more detail with reference to Figure 2A. As shown schematically in Figure 2A, the container 4 is configured to contain a volume of medicament M. At its proximal end, the container 4 is in fluid communication with fluid path 6, which delivers fluid to a needle (not shown). The fluid line may comprise a conduit coupling the proximal end of the container 4 to a separate needle. Alternatively, the container 4 may be directly connected to a needle, such that the fluid line 6 is provided by the needle without intermediate components. The container 4 is closed at its distal end with stopper 4a.

The drive system 10 is configured to expel medicament from the container 4 by advancing stopper 4a axially along the container body 4' towards the proximal end of the container 4. The drive system comprises an axial drive component configured to bear upon the stopper 4a of the cartridge, and a variable rate drive configured to drive movement of the axial drive component.

In the embodiment illustrated in Figure 2A, the drive system comprises a telescopic screw assembly 102 configured to engage the stopper 4a and drive it axially along the container 4 to discharge medicament from the container 4. The telescopic screw assembly 102 can engage the stopper 4a directly, or an intermediate component, such as a piston rod, may be provided between the telescopic screw assembly 102 and the stopper 4a. The telescopic screw assembly 102 comprises a first part 102a in threaded engagement with a second part 102b. Rotation of the second part 102b relative to the first part 102a causes linear displacement of the first part 102a relative to the second part 102b. The linear displacement of the first part 102a relative to the second part 102b advances the stopper 4a along the container body4' to deliver the medicament M. In the embodiment shown in Figure 2A, the rotating component of the telescopic screw assembly 102 is shown with reference numeral 102b. The part of the telescopic screw assembly 102 that acts as the axial drive component is labelled with reference numeral 102a. For convenience, in the following description of illustrative embodiments, the invention will be described with reference to a telescopic screw assembly 102 shown in Figure 2A. However, the skilled person will appreciate that the telescopic screw assembly may be replaced with an alternative arrangement for driving axial movement of the axial drive component 102a.

The drive system 10 also includes a variable rate drive 104 comprising a motor 104a configured to drive the telescopic screw assembly 102. The motor 104a can be configured to drive rotation of the screw assembly parts using any suitable engagement. For example, the motor 104a may be coupled to the telescopic screw assembly 102 by any suitable gear assembly. As shown in Figure 1, the telescopic screw assembly 102 can be arranged within the housing 2 adjacent to the drive unit 10, with a gear assembly 106 arranged between the motor 104a and the telescopic screw assembly 102. However, it will be appreciated that the telescopic screw assembly 102 can be replaced with other drive components. For example, in some embodiments, the motor 104a may use a rack and pinion arrangement to drive linear motion of a drive component.

The motor 104a can be any suitable type of motor as known in the art. For example, the motor 104a can be a constant power motor or a pulse width modulation (PWM) controlled motor. In an exemplary embodiment, the motor 104a may be a PWM controlled motor, wherein the speed of the motor 104a is controlled by varying the duty cycle of the PWM signal that drives the motor 104a. Varying the speed of the motor 104a, varies the speed of advancement of the stopper 4a along the container body 4', and thus the rate at which medicament M is delivered to the injection site through the needle.

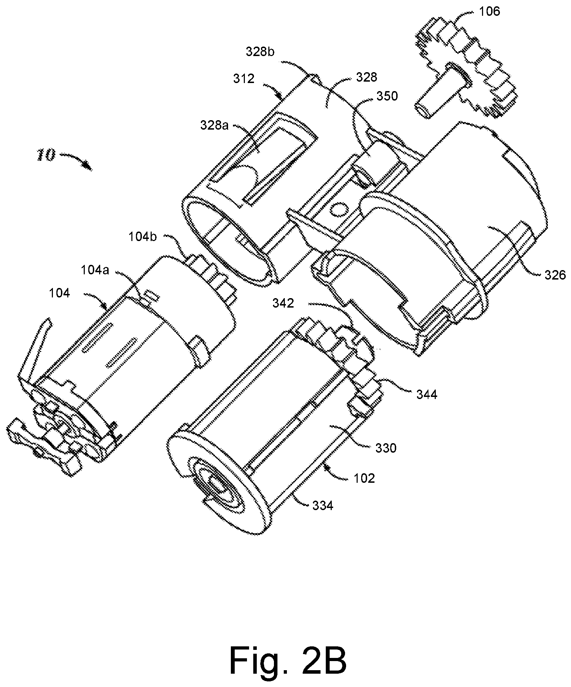

Fig. 2B shows one exemplary drive system configuration that may be used in the present invention. As shown in Fig. 2B, the drive system 10 comprises a chassis 312, configured to be secured within the housing of the injector. The variable rate drive 104, telescoping driving assembly 102, and a transmission gear 106 (shown schematically in Figure 2A) are also shown in Figure 2B.

The variable rate drive 104 and the telescoping driving assembly 102 are mounted within the chassis 312. In the illustrated embodiment, the variable rate drive 104 takes the form of a motor 104a, but the present disclosure is not so limited. For example, the variable rate drive 104 may take the form of an electric motor, an electromechanical motor, combinations thereof, or the like. The variable rate drive 104 drives expansion of the telescoping driving assembly 102 and is connected to the telescoping driving assembly 102 via the transmission gear 106.

The chassis 312 includes a first sleeve 326 and a second sleeve 328 generally parallel to the first sleeve 326, with a third sleeve 350 therebetween. The telescoping driving assembly 102 is slidably received within the first sleeve 326, the variable rate drive 104 is slidably received within the second sleeve 328 and the transmission gear 106 is mounted to the third sleeve 350. The second sleeve 328 generally corresponds in size and shape to the variable rate drive 104. In the illustrated embodiment, the variable rate drive 104 and the second sleeve 328 include complementary, i.e., reciprocal, snap connection components 314a, 328a, respectively, to slidably secure and lock the variable rate drive 104 into the second sleeve 328.

The second sleeve 328 also includes a stop member 328b at a rear end thereof to abut the variable rate drive 104 when inserted. In the illustrated embodiment, the stop member 328b takes the form of a radially inwardly extending lip, but the disclosure is not so limited. Advantageously, no additional tools are required for securing the variable rate drive 104 within the second sleeve 328.

Turning to the telescoping driving assembly 102, the telescoping driving assembly 102 is configured to engage and advance the piston through the cartridge to expel the substance out of the cartridge. One non-limiting example of a telescoping driving assembly 102 is described in U.S. Patent Application No. 14/725,009, entitled, "Linear Rotation Stabilizer For A Telescoping Syringe Stopper Driver Assembly".

The second sleeve 328 of the chassis 312 defines at least partially open front and rear ends, such that the variable rate drive 104 secured therein may engage other operational components at either end. A variable rate drive gear 104b of the variable rate drive 104, positioned adjacent the rear end of the second sleeve 328 when the variable rate drive 104 is secured within the sleeve 328, engages the transmission gear 106. The shaft (linear component of the telescopic drive assembly 102a) is operatively engaged with a telescoping assembly rotation gear 344 adjacent the inner closed end of the collar 342. The transmission gear 106 engages the rotation gear 344 via an opening in the first sleeve 326. Accordingly, the variable rate drive 104 is operatively engaged with the telescoping driving assembly 102.

In operation, the variable rate drive 104, the telescoping driving assembly 102 and the transmission gear 106 are secured within the chassis 312 in the manner previously disclosed. When secured to the chassis 312, the variable rate drive 104 is operatively engaged with the telescoping driving assembly 102 such that rotation of the variable rate drive gear 104b drives the telescoping driving assembly 102. The telescoping driving assembly 102 is aligned with a bay/track for the cartridge 4 when the drive assembly 10 is secured to the housing of the drug delivery device. Once the cartridge 4 is inserted in the bay/track, and the injector is activated, the variable rate drive 104 drives the telescoping driving assembly 102 to expand, engage and advance the piston 4a through the cartridge 4 to expel substance therefrom.

A controller 108 controls the speed of the motor that drives the variable rate drive 104. The motor 104a can be controlled using an open loop control system, wherein the speed at which the stopper 4a advances is estimated based on the voltage applied to the motor 104a. However, it is preferred that the motor 104a is controlled using a closed loop system in which the motion of the motor or another drive system component (e.g. the telescopic screw assembly) is measured, the rate of injection determined and fed back to controller 108, and the applied voltage (or the duty cycle for a PWM controlled motor) varied to maintain the desired advancement of the stopper 4a.

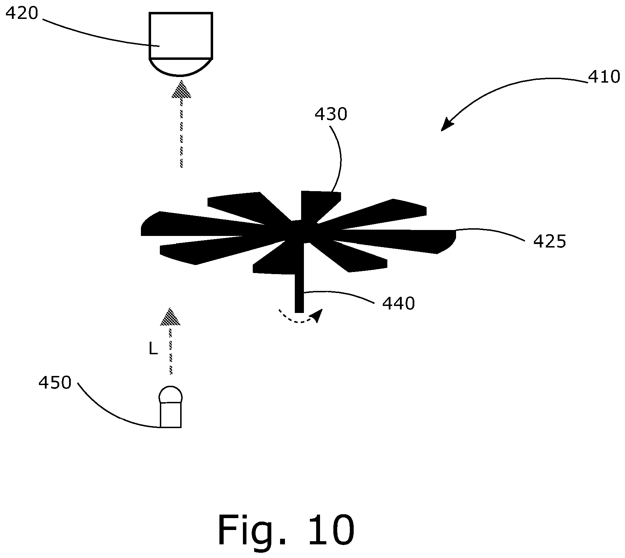

In one exemplary embodiment, the speed of the motor 104a is measured directly using an optical encoder (an example of an optical encoder is described below in relation to Fig. 10). The encoder comprises a photosensor and a light source. A rotating component 110 coupled to the rotor of motor 104a is positioned between the photosensor and the light source. The rotating component may comprise one or more blades, or a disk having one or more apertures.

In at least one exemplary embodiment, the encoder comprises at least 4 blades, advantageously 8 blades. Increasing the number of blades of the of the encoder increases the precision with which the position of the rotor can be determine, and therefore increases the precision with which the position of the axial drive component is known. It will be appreciated that the gearing between the motor 104a and the telescopic screw assembly can also increase the precision with which the position of the drive component is known because the distance travelled by the drive component 102a for each turn of the rotor can be varied by varying the gear assembly between the motor and the axially advancing drive component. The particular configuration of the optical encoder 410 and the coupling between the motor 104a and the axially advancing drive component 102a can be selected based on the motor selected, the dosage regime required, the volume of the container, and other parameters, as will be understood by a person skilled in the art in light of the present disclosure.

The drive system 10 is configured to control the speed of the motor 104a to deliver one or more dosages of medicament at a controlled rate. In at least one exemplary embodiment, the controller 8 is configured to control the variable rate drive to deliver a first pre-defined volume V1 of medicament from the container 4 at a first rate R1, and a second predefined volume V2 of medicament at a second rate R2.

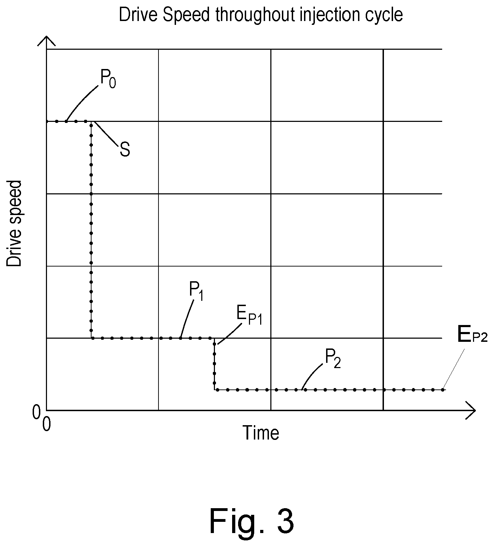

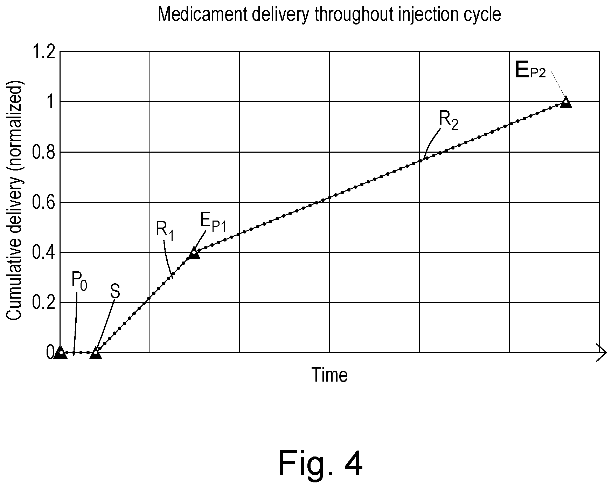

To control the delivery rate of the first and second volumes V1, V2, the controller 108 comprises a processor 112 configured with reference to Figs. 3 and 4: (i) to identify the start S of injection by identifying completion of a pre-determined preparatory step or event P0; (ii) control the variable rate drive to run at a first speed during a first phase P1 from the identified start point to deliver the first volume V1; (iii) identify an end point E_{P1} for the first phase P1; and (iv) control the variable rate drive to run at a second speed for a second phase P2 to deliver the second volume V2. The controller 108 may also be configured to determine an end point E_{P2} for the second phase P2 and control the drive to stop when E_{P2} has been identified. However, for bi-phasic delivery devices (comprising only two drug delivery phase P1, P2) the end point of the second phase E_{P2} can be defined as the end of injection when the container 4 is empty. In all embodiments of the invention, end wall detection may be employed to prevent the device from bearing upon the proximal end wall of the container 4 once all medicament has been expelled and the container 4 is empty.

As discussed above, the control system is preferably configured to control the speed of the motor 104a using a closed loop system, in which the speed of advancement of the axial drive component 102a is measured (directly or indirectly) and the duty cycle of a PWM controlled motor varied accordingly to deliver the drug at the prescribed rate.

The end of the first delivery phase P1 can be determined in different ways. For example, the end of the first delivery phase P1 can be determined as the expiry of a predetermined time period. Since the speed of the motor is known, the distance travelled by the axial drive component 102a can be calculated, and the expiry of the predefined time period can coincide with completion of delivery of the first volume V1.

In at least one embodiment, the end of the first delivery phase P1 can be determined as the completion of a predetermined travel distance of the axial drive component. This can be measured directly by measuring advancement of the axial drive component or it can be calculated based on the measured speed and position of the motor 104a, as measured by the optical encoder 410.

The pre-determined preparatory step P0 or event that is used to identify the start time of injection can be a predetermined number of rotations of the motor 104a (corresponding to an estimated travel distance of the axial drive component or telescopic screw assembly 102).

In at least one embodiment, the controller 108 is configured to count pulses in the signal output from the optical encoder 410 to determine a number of rotations of the motor. For example, the optical encoder 410 can be configured such that eight pulses in the output signal corresponds to one complete revolution of the motor. Therefore, the predetermined event that identifies the start of injection may be a predetermined number of pulses in the output signal of the optical encoder 410 as counted by the controller.

At a predetermined number of rotations of the motor, the distance travelled by the axial drive component 102a can be calculated, and the point at which the axial drive component 102a is calculated to have reached the stopper 4a can be deemed the start of drug delivery. The predetermined number of rotations of the motor 104a that is used as a parameter for estimating a start point for injection can be varied depending on, for example, the container 4 inserted into the device and the fill level of the container 4.

The end of the first delivery phase P1 may be identified in a similar manner. For example, the end of the first delivery phase, when volume V1 has been delivered, may be determined based on the distance travelled by the axial drive component 102a. This can be measured directly or may be calculated based on measured rotations of the motor 104a, as measured by the optical encoder. Since the measured pulses are representative of the distance travelled by the axial drive component 102a, and the distance travelled by the axial drive component defines the volume of medicament delivered, delivery of the volume V1 can be deemed complete after a predetermined number of pulses have been counted after the identified start time for drug delivery.

In alternative embodiments, the determination of the start of injection and the end of phase P1 can be carried out in different ways. For example, the position of the axial drive component can be measured directly (rather than calculated based on the rotation of the motor 104a). Other means of determining the start point of injection and/or the end of phase P1 may also be used.

It will be appreciated that in many drug delivery devices and systems, a drive system may advance in an 'idle' phase before the axial drive component 102a is brought into engagement with the plunger or stopper 4a of a container 4. During this phase, although the motor 104a drives movement of the axial drive component 102a, axial movement of the drive does not result in axial movement of the stopper 4a until any gap between the axial drive component 102a and the stopper 4a is closed. As such no drug is delivered from the container 4 during the idle phase. For a medicament delivered in a single bolus at a single rate, the drive can be driven at a constant speed throughout the idle phase and the delivery phase of drive advancement.

Alternatively, for a single rate infusion device, the drive can be driven at relatively high speed (e.g. maximum speed) during the idle phase and a lower delivery speed during delivery of medicament. In the event that a motor fault occurs in such a device during the idle phase, the speed of the drive will be slower than expected during the idle phase, and delivery of medicament may begin later than expected due to the drive advancing for a longer period than expected without acting upon the stopper 4a. The outcome of such a fault may be that the start of drug delivery is delayed, but the rate of delivery of the bolus of medicament once started will be according to the prescribed rate.

For a multi-rate delivery device, for example a bi-phasic delivery device (e.g. in which the drive is driven at a first rate R1 until a first volume V1 is delivered and a rate R2 until a second volume V2 has been delivered), a delay to the start of drug delivery may lead to an unacceptable drug delivery profile because the rate at which the drug is delivered by the device may be incorrect. For example, in a device configured to switch from R1 to R2 upon expiry of a prescribed time period, an unknown delay in the start of drug delivery would lead to a smaller volume of drug being delivered at rate R1, and a larger volume of drug being delivered at rate R2. However, for a device configured to deliver a volume V1 before the end of a prescribed time period, a delay to start of injection would lead to R1 being higher than prescribed to ensure that the volume V1 was delivered before the end of the prescribed time period. In either case, the prescribed drug delivery profile is not provided by a device encountering a motor fault which leads to a delay to the estimated start of injection.

By contrast, for drive systems configured according to the present disclosure, the progress of drug delivery is determined by calculating the advancement of the axial drive component based on the number of turns completed by the motor. In this manner, start point of injection can be determined by determining completion of a predefined event (e.g. completion of a predefined number of motor turns), and the rate of drug delivery R1 and R2 can be closely controlled by the drive system because the controller monitors the speed of the motor, calculates the rate of advancement of the axial drive component, and can thus closely control the speed of advancement of the stopper 4a.

It will be appreciated that, in some embodiments, the idle movement of the drive (before the axial drive component is brought into engagement with the stopper 4a) may be driven at a third speed, which can be higher than the first and second speeds used to deliver V1 and V2 of medicament. In such embodiments, the drive system can be further configured to control the variable rate drive to run at a third speed until the predefined preparatory step P0 has been completed. Since movement of the drive during the idle phase does not drive movement of the stopper 4a, the motor 104a can be run at high speed (compared to the motor speeds used for drug delivery). In some embodiments, the motor 104a may be driven at maximum speed, for example 100% duty cycle.

Accordingly, in some embodiments, the control system can be configured to control the drive to move at three different speeds: at speed S1, before the start point at which delivery of medicament is identified as having begun; at speed S2, for delivery of medicament at a first rate R1; and at speed S3, for delivery of medicament at a second rate R2.

It will be appreciated that in the context of the present disclosure, the predetermined rate (R1, R2, ...Rn) at which medicament is delivered is a set rate, determined and maintained by the controller. The set rate may be constant or substantially constant to allow continuous drug delivery. In embodiments of the invention, the device may be configured to provide substantially continuous drug delivery, without (or with de minimis) interruption of drug delivery during a drug delivery cycle. Figure 3 shows the speed of the motor (or another drive component) throughout an exemplary injection cycle. As shown in Figure 3, the telescopic screw assembly 102 is driven at a first speed S1 during a first phase P0, until the start point S of the injection is identified. The first phase P0 can be defined as the period before a predetermined number of motor revolutions has occurred.

After the start point for drug delivery has been identified (e.g. the predetermined number of turns has been reached), the motor 104a is driven at a second lower speed, to ensure that a second travel distance of the drive (corresponding to V1) is achieved during the second phase P1 at the first prescribed rate R1. A second drug delivery phase P2 begins when the second travel distance has been completed, after which point the motor is driven at a third speed, to ensure that a second travel distance (corresponding to V2) is completed during the second phase P2 at the second prescribed rate R2.

Figure 4 shows the rate of drug delivery over time delivered by the drive speed profile illustrated in Figure 3. As shown in Figure 4, no drug is delivered during a first travel distance of the drive during the first phase P0 because the telescopic drive assembly 102 is not engaged with the stopper 4a. However, from the identified start point of drug delivery, medicament is delivered at a first rate during phase P1, and at a second rate during phase P2.

In the embodiment illustrated in Figures 3 and 4, the injection ends at the end of the second phase P2. The illustrated embodiment from Figures 3 and 4 therefore provides bi-phasic drug delivery, delivering a first volume of medicament V1 at a first rate R1, and a second volume of medicament V2 at a second rate R2, whereupon V1+V2= V_{TOTAL} such that all medicament contained in the container 4 has been delivered by the end of phase P2.

It will be appreciated however, that the present invention is not limited to the delivery rates or dosage regime illustrated in Figures 3 and 4. Rather, the control system may be configured to deliver a third volume of medicament in a third phase at a third rate, and optionally fourth, fifth,.. *n*th volumes of medicament may be delivered at fourth, fifth,... *n*th rates respectively. The periods, volumes and rates can be varied according to the dosage regime required for a given medicament, and/or a given patient.

In an exemplary embodiment, the device can be adapted to deliver first and second volumes of medicament V1 and V2 over a period of several minutes or hours. For example, in the embodiment shown in Figures 3 and 4, a total medicament delivery period T_{D} can be defined as a time taken to complete the first phase P1 and a time taken to complete the second phase P2 and wherein T_{D} is at least 2.5 minutes, at least 5 minutes, at least 10 minutes, at least 30 minutes, at least 60 minutes, at least 120 mins, at least 240 minutes; or at least 300 minutes.

It will be appreciated that the number of delivery phases, the volume of drug delivered in each phase, and the rate at which each volume is delivered can be varied beyond the embodiment illustrated in Figures 3 and 4 and can be adapted to any dosage regime required.

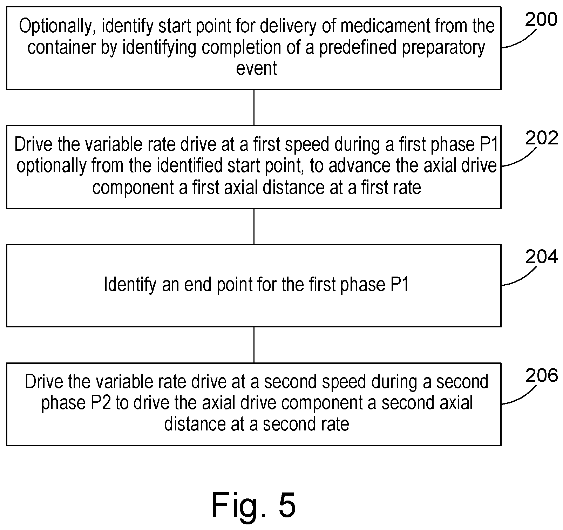

Referring now to Figure 5, a method of controlling a drive system according to the disclosure will be described. The drive system 10 comprises an axial drive component 102a configured to drive advancement of a stopper 4a sealing a container 4, a variable rate drive 104 configured to drive advancement of the axial drive component 102a, and a controller 108 configured to drive the variable rate drive, as described above. As illustrated in Figure 5, the method comprises a first step 200 of identifying start point for delivery of medicament from the container by identifying completion of a predefined preparatory event. Once the start point of drug delivery has been identified, the controller drives the variable rate drive at a first speed during a first phase P1 from the identified start point to advance the axial drive component a first axial distance at a first speed 202. At step 204, the controller identifies an end point for the first phase P1 before driving, at step 206, the variable rate drive at a second speed during a second phase to advance the axial drive component a second axial distance at the second speed. For a multi-rate delivery device, the first speed is different to the second speed. The first and second volumes may be the same or may be different, and the time taken to complete the first and second phases may be different or may be the same.

The predetermined preparatory event or step P0 used to identify the start of drug delivery can be completion of a predetermined distance travelled by the axial drive component. This can be measured directly, or may be calculated based on measured rotations of the motor. The rotation of the motor may be measured using an optical encoder. In at least one embodiment, the controller 108 is configured to count pulses in the signal output from the optical encoder to determine a number of rotations of the motor. For example, the optical encoder can be configured such that eight pulses in the output signal corresponds to one complete revolution of the motor. Therefore, the predetermined event that identifies the start of injection may be a predetermined number of pulses in the output signal of the optical encoder as counted by the controller.

The end of the first delivery phase may be identified in a similar manner. For example, the end of the first delivery phase, when volume V1 has been delivered, may be determined based on the distance travelled by the axial drive component 102a. This can be measured directly, or may be calculated based on measured rotations of the motor 104a, as measured by the optical encoder. Since the measured pulses are representative of the distance travelled by the axial drive component 102a, and the distance travelled by the axial drive component defines the volume of medicament delivered, delivery of the volume V1 can be deemed complete after a predetermined number of pulses have been counted after the identified start time for drug delivery.

Since some or all of the progress of a drug delivery process can be monitored and controlled by counting pulses in the encoder signal, which is representative of the number of motor turns completed, the drive system can be modified to suit a number of different container fill levels, and dosage regimes, by adjusting the parameters by which the controller uses to identify the start point for drug delivery and the end point for each phase of injection. This can allow the device to be adapted to a number of different drug delivery profiles without changes in hardware or software. Instead, the pre-set number of pulses for each phase of an injection cycle can be modified to adapt for different cartridge fill levels, different dosage profiles, etc.

Moreover, the closed loop control system for the motor, and the direct measurement of motor progress can provide precise control of the movement of the axial drive component and thus the progress of drug delivery. Moreover, by controlling the rate of drug delivery in this manner, the devices and methods are robust against common motor faults.

In some embodiments, the step of identifying a start point for delivery of medicament by identifying completion of a predetermined event may be omitted. For example, for devices in which it is known (or can be assumed) that idle advancement of the axial drive component has been eliminated, or for devices in which the idle travel is negligible relative to the total travel of the axial drive component, it can be assumed that the start point for delivery of medicament is the start point for travel of the axial drive component. In such embodiments, the step of identifying a start point for delivery can be omitted entirely, and the drive systems and methods described herein can be used to control the speed of the drive (and the rate of drug delivery) for the first and second volumes V1, V2 of medicament. The endpoint E_{P1} for the first delivery phase can be identified as described above, and steps 202, 204 and 206 (as shown in Figure 5) carried out as described above.

The controller 108 described above in connection with the systems and methods according to the invention may be further configured to store encoder signals and the power requirements of the motor for diagnostic purposes.

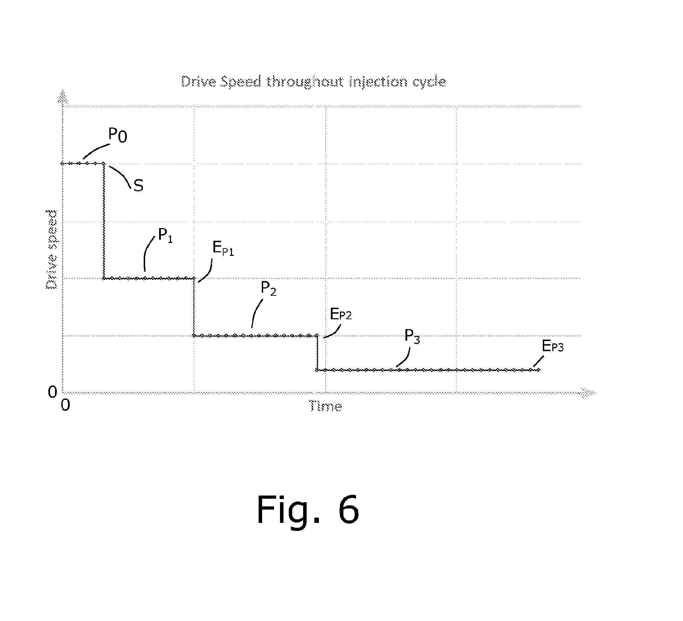

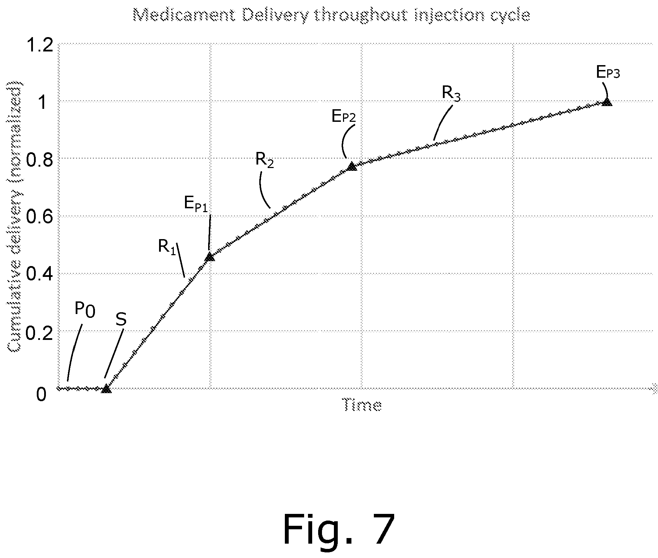

Referring now to figures 6 and 7, a control system configured to deliver a first volume, a second volume, and a third volume of medicament is shown. The illustrated embodiment provides a tri-phasic drug delivery, delivering a first volume of medicament V1 at a first rate R1, a second volume of medicament V2 at a second rate R2, and a third volume of medicament V3 at a third rate R3, whereupon V1+V2+V3=VTOTAL such that all medicament contained in the container has been delivered by the end of phase P3. In the illustrated embodiment, the first rate R1 is greater than the second rate R2 which is, in turn, greater than the third rate R3. Although, the rates R1, R2, and R3 may not decrease at each phase. For example, the second rate R2 may be greater than the first rate **R1,** and the third rate R3 may be less than the second rate R2.

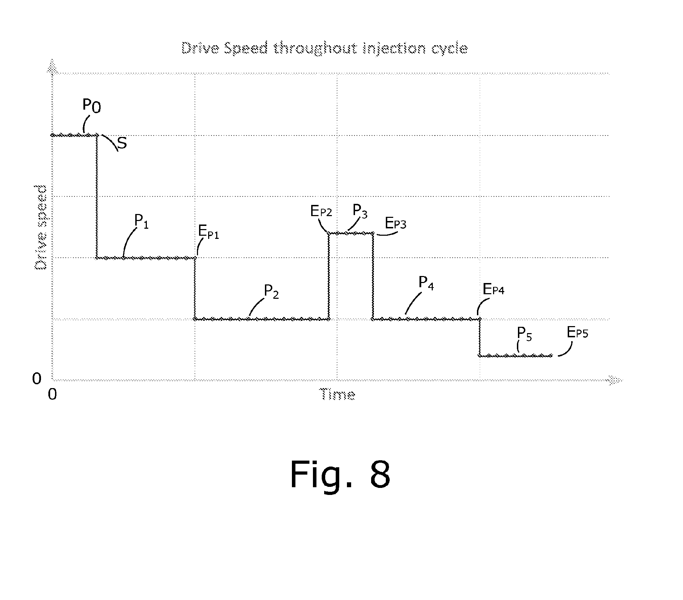

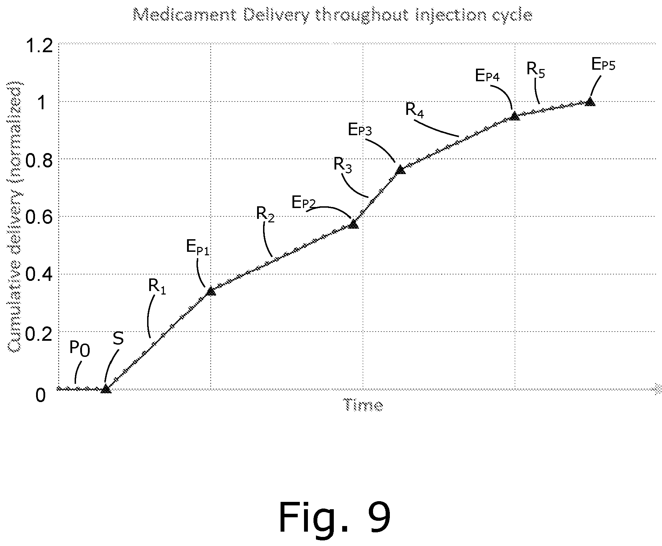

Figures 8 and 9 show a control system configured to deliver a first volume, a second volume, a third volume, a fourth volume, and a fifth volume of medicament. The illustrated embodiment provides a penta-phasic drug delivery, delivering a first volume of medicament V1 at a first rate **R1,** a second volume of medicament V2 at a second rate R2, a third volume of medicament V3 at a third rate R3, a fourth volume of medicament V4 at a fourth rate R4, and a fifth volume of medicament V5 at a fifth rate R5, whereupon V1+V2+V3+V4+V5=VTOTAL such that all medicament contained in the container has been delivered by the end of phase P5. In the illustrated embodiment the second rate R2 is less than the first rate **R1,** the third rate R3 is greater than the first rate **R1,** the fourth rate R4 is less than the third rate R3, and the fifth rate R5 is less than the fourth rate R4. The skilled person would understand that the rates may be chosen in any of the five phases to fit a desired delivery profile.

An optical encoder 410, is shown in Figure 10, and may comprise a fixed optical sensor 420 and a rotating encoder wheel 425, the encoder wheel including at least one blocking arm 430. The at least one blocking arm, may be a plurality of blocking arms, for example two blocking arms, 6 blocking arms, 8 blocking arms, **11** blocking arms, or any other positive integer number of blocking arms. The fixed optical sensor 420 is configured to receive light emitted from a light source 450. The light source may, for example, be a light emitting diode, a bulb, or any other light source. The at least one blocking arm or the plurality of blocking arms are configured to rotate about an axis 440 in coordination with a rotating part of a drug delivery device. As the at least one blocking arm or plurality of blocking arms rotate they move into and out of the path L of the light emitted from light source 450 and received by the fixed optical sensor 420. When the light path L is blocked a signal indicates that the light is not being received by the fixed optical sensor. When the blocking arm moves out of the light path, a signal from the optical sensor indicates that light is being received by the optical sensor. As such, a series of pulses are produced by the fixed optical sensor indicating the changing angular position of the encoder wheel.

The fixed optical sensor may be configured to generate a signal which may be processed by the controller to determine that the encoder wheel is rotating. In the case of a single arm on the encoder wheel, each pulse received at the controller from the fixed optical sensor indicates that the rotating encoder wheel has rotated a full turn of 360 degrees. This in turn indicates that a rotating part of the delivery device has also rotated a full turn. In the case that there are a plurality of blocking arms then a corresponding plurality of pulses indicates a full turn of the encoder wheel and the rotating part of the delivery device.
In alternative arrangements, the light source may be located on the same side of the encoder wheel as the fixed optical sensor. In such an arrangement, the encoder wheel is configured, in segments, to either reflect light into the fixed optical sensor or not reflect the light into the optical sensor.

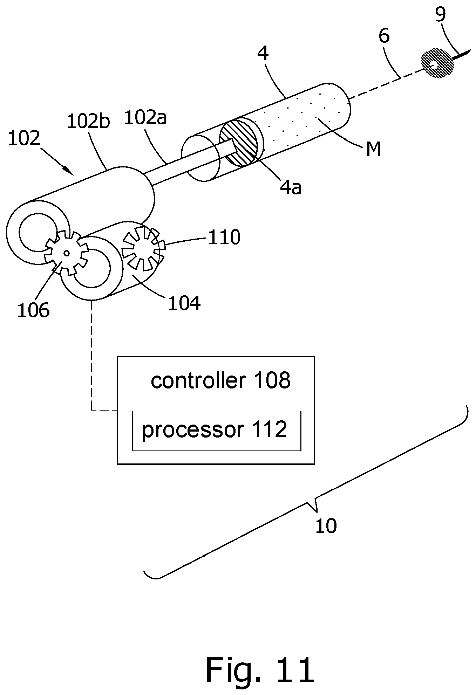

Figure 11 shows a schematic view of a drive system 10 similar to the drive system 10 of Figure 2A, coupled with a container 4. At its proximal end, the container 4 is in fluid communication with a fluid path 6, which delivers fluid to a needle 9.

Further advantages provided by the present systems and methods will be apparent to the skilled person in light of the present disclosure.

The preceding detailed description describes drive systems and methods in combination with an exemplary drug delivery device. However, the skilled person will understand that the invention is not limited to use in connection with the exemplary device described herein. Rather, one or more benefits associated with the present invention may be implemented in connection with other drug delivery devices, as will be apparent to the skilled person in light of the preceding detailed description.

Steps carried out by the controller and described in combination with the inventive methods disclosed herein can be carried out by devices described as incorporating controller 108. Similarly, functionality described in combination with the drive system 10 and the exemplary drug delivery device may form part of the inventive methods described herein.

It will also be understood that, where used, terms such as "proximal", "distal", "front", "back", "side", "top" and "bottom" are used for convenience interpreting the drawings and are not to be construed as limiting. The term "comprising" should be interpreted as meaning 'including but not limited to', such that it does exclude the presence of features not listed.

The embodiments described above are provided as exemplary embodiments of the invention to illustrate one or more ways in which the invention may be put into effect. The embodiments described and shown in the accompanying drawings are not intended to be limiting on the scope of the invention, and modification may be made, and elements may be replaced with functionally or structurally equivalent parts, and features of different embodiments may be combined without departing from the scope of the invention.

## Claims

1. A drug delivery device comprising:
a housing (2) configured to receive a container (4) containing a volume of medicament;
an actuator (8) configured to initiate a drug delivery procedure; and
a drive system (10) comprising:
an axial drive component (102, 102a, 102b) configured to drive advancement of a stopper (4a) sealing the container (4);
a variable rate drive (104) configured to drive advancement of the axial drive component (102, 102a, 102b), wherein the variable rate drive (104) comprises a motor (104a) configured to drive the axial drive component; and
a controller (8, 108) comprising a processor (112),
**characterized in that** the controller (8, 108) is configured to:
a. drive the variable rate drive (104) at a first speed during a first phase P1 to deliver a first predefined volume V1 of the medicament from the container (4) at a first rate R1;
b. identify an end point for the first phase P1 by identifying completion of a predetermined number of rotations of the motor (104a); and
c. drive the variable rate drive (104) at a second speed during a second phase P2 to deliver a second predefined volume V2 of the medicament from the container (4) at a second rate R2.

2. The drug delivery device according to claim 1, wherein the controller (8, 108) is further configured to identify a start point for delivery of medicament from the container (4), wherein the start point is identified by identifying completion of a predefined preparatory event, and wherein the first phase P1 begins at the start point.

3. The drug delivery device according to claim 1 or claim 2, wherein the axial drive component (102, 102a, 102b) is part of a telescopic screw assembly (102) of the drive system.

4. The drug delivery device according to any preceding claim, wherein the motor (104a) is a pulse width modulation (PWM) driven motor (104a).

5. The drug delivery device according to any of claims 2 to 4, wherein the predetermined preparatory event is one of:
completion of a predetermined distance travelled by the axial drive component (102, 102a, 102b);
completion of a predetermined preparatory number of motor (104a) rotations; and
completion of a pre-determined time-period.

6. The drug delivery device according to any preceding claim, wherein the drive system (10) comprises an optical encoder (410) to monitor a speed of the motor (104a) and provide feedback to the controller (8, 108).

7. The drug delivery device according to claim 6, wherein the controller (8, 108) is configured to adjust power supplied to the motor (104a) in response to the monitored speed of the motor (104a).

8. The drug delivery device according to any preceding claim, wherein the controller (8, 108) is configured to control the variable rate drive (104) to run at a preparatory speed during a preparatory phase P0 until completion of the predetermined preparation event.

9. The drug delivery device according to claim 8, wherein the preparatory speed is greater than the first speed S1 and the second speed S2.

10. The drug delivery device according to any preceding claim, wherein a medicament delivery period (T_{D}) is defined as a time taken to complete the first phase (T_{P1}) and a time taken to complete the second phase (T_{P2}), and wherein the medicament delivery period (T_{D}) is:
at least 2.5 minutes;
at least 5 minutes;
at least 10 minutes;
at least 30 minutes;
at least 60 minutes;
at least 120 mins;
at least 240 minutes; or
at least 300 minutes.

11. The drug delivery device according to any one of claims 1 to 9, wherein the controller (8, 108) is further configured to drive the variable rate drive (104) to deliver a third predefined volume V3 of the medicament from the container (4) at a third rate R3, and
wherein the controller (8, 108) is configured to:
a. identify an end point for the second phase P2; and
b. drive the variable rate drive (104) at a third speed during a third phase P3, to deliver the third predefined volume V3 of the medicament at the third rate R3.

12. The drug delivery device according to any one of claims 1 to 10, wherein the controller (8, 108) is configured to drive the variable rate drive (104) to deliver a tuple predefined volume Vn of the medicament from the container (4) at a tuple rate Rn, and
wherein the controller (8, 108) is configured to:
a. identify an end point for a phase Pn-1 before a tuple phase Pn; and
b. drive the variable rate drive (104) at a tuple speed during the tuple phase Pn to deliver the tuple predefined volume Vn of the medicament at the tuple rate Rn.

13. The drug delivery device according to any of the preceding claims, wherein the second rate R2 is less than the first rate R1.

## Patentansprüche

1. Medikamenten-Verabreichungsvorrichtung, umfassend:
ein zur Aufnahme eines ein Medikamentenvolumen enthaltenden Behälters (4) ausgestaltetes Gehäuse (2),
einen zur Einleitung eines Medikamentenverabreichungsvorgangs ausgestalteten Aktuator (8) und
ein Antriebssystem (10), das Folgendes umfasst:
eine axiale Antriebskomponente (102, 102a, 102b), die zum Antrieb des Vorschiebens eines den Behälter (4) abdichtenden Stopfens (4a) ausgestaltet ist,
einen Antrieb (104) mit variabler Geschwindigkeit, der dazu ausgestaltet ist, den Vorschub der axialen Antriebskomponente (102, 102a, 102B) anzutreiben, wobei der Antrieb (104) mit variabler Geschwindigkeit einen zum Antrieb der axialen Antriebskomponente ausgestalteten Motor (104a) umfasst, und
eine Steuerung (8, 108), die einen Prozessor (112) umfasst,
**dadurch gekennzeichnet, dass** die Steuerung (8, 108) dazu ausgestaltet ist:
a. den Antrieb (104) mit variabler Geschwindigkeit während einer ersten Phase P1 mit einer ersten Geschwindigkeit anzutreiben, um ein erstes vordefiniertes Volumen V1 des Medikaments mit einer ersten Rate R1 aus dem Behälter (4) zu verabreichen,
b. einen Endpunkt für die erste Phase P1 durch Identifizieren des Abschlusses einer vorbestimmten Anzahl von Umdrehungen des Motors (104a) zu identifizieren und
c. den Antrieb (104) mit variabler Geschwindigkeit während einer zweiten Phase P2 mit einer zweiten Geschwindigkeit anzutreiben, um ein zweites vordefiniertes Volumen V2 des Medikaments mit einer zweiten Rate R2 aus dem Behälter (4) zu verabreichen.

2. Medikamenten-Verabreichungsvorrichtung nach Anspruch 1, wobei die Steuerung (8, 108) ferner dazu ausgestaltet ist, einen Startpunkt für die Verabreichung eines Medikaments aus dem Behälter (4) zu identifizieren, wobei der Startpunkt durch Identifizieren des Abschlusses eines vordefinierten vorbereitenden Ereignisses identifiziert wird und wobei die erste Phase P1 an dem Startpunkt beginnt.

3. Medikamenten-Verabreichungsvorrichtung nach Anspruch 1 oder Anspruch 2, wobei die axiale Antriebskomponente (102, 102a, 102b) Teil einer Teleskopschraubenbaugruppe (102) des Antriebssystems ist.

4. Medikamenten-Verabreichungsvorrichtung nach einem der vorhergehenden Ansprüche, wobei der Motor (104a) ein durch Pulsbreitenmodulation (PBM) angetriebener Motor (104a) ist.

5. Medikamenten-Verabreichungsvorrichtung nach einem der Ansprüche 2 bis 4, wobei das vorbestimmte vorbereitende Ereignis eines von Folgenden ist:
Abschluss einer von der axialen Antriebskomponente (102, 102a, 102b) zurückgelegten vorbestimmten Strecke,
Abschluss einer vorbestimmten vorbereitenden Anzahl von Umdrehungen des Motors (104a) und
Abschluss einer vorbestimmten Zeitspanne.

6. Medikamenten-Verabreichungsvorrichtung nach einem der vorhergehenden Ansprüche, wobei das Antriebssystem (10) einen optischen Codierer (410) umfasst, um eine Drehzahl des Motors (104a) zu überwachen und eine Rückmeldung an die Steuerung (8, 108) bereitzustellen.

7. Medikamenten-Verabreichungsvorrichtung nach Anspruch 6, wobei die Steuerung (8, 108) dazu ausgestaltet ist, als Reaktion auf die überwachte Drehzahl des Motors (104a) die dem Motor (104a) zugeführte Leistung zu justieren.

8. Medikamenten-Verabreichungsvorrichtung nach einem der vorhergehenden Ansprüche, wobei die Steuerung (8, 108) dazu ausgestaltet ist, den Antrieb (104) mit variabler Geschwindigkeit so zu steuern, dass er während einer Vorbereitungsphase P0 bis zum Abschluss des vorbestimmten Vorbereitungsereignisses mit einer vorbereitenden Drehzahl läuft.

9. Medikamenten-Verabreichungsvorrichtung nach Anspruch 8, wobei die Vorbereitungsdrehzahl größer als die erste Drehzahl S1 und die zweite Drehzahl S2 ist.

10. Medikamenten-Verabreichungsvorrichtung nach einem der vorhergehenden Ansprüche, wobei ein Medikamentenverabreichungszeitraum (T_{D}) als eine Zeit definiert ist, die zum Abschluss der ersten Phase (T_{P1}) benötigt wird, und eine Zeit, die zum Abschluss der zweiten Phase (T_{P2}) benötigt wird, und wobei der Medikamentenverabreichungszeitraum (T_{D}) wie folgt ist:
mindestens 2,5 Minuten,
mindestens 5 Minuten,
mindestens 10 Minuten,
mindestens 30 Minuten,
mindestens 60 Minuten,
mindestens 120 Minuten,
mindestens 240 Minuten oder
mindestens 300 Minuten.

11. Medikamenten-Verabreichungsvorrichtung nach einem der Ansprüche 1 bis 9, wobei die Steuerung (8, 108) ferner dazu ausgestaltet ist, den Antrieb (104) mit variabler Geschwindigkeit anzutreiben, um ein drittes vordefiniertes Volumen V3 des Medikaments aus dem Behälter (4) mit einer dritten Rate R3 zu verabreichen, und
wobei die Steuerung (8, 108) dazu ausgestaltet ist:
a. einen Endpunkt für die zweite Phase P2 zu identifizieren und
b. den Antrieb (104) mit variabler Geschwindigkeit während einer dritten Phase P3 mit einer dritten Geschwindigkeit anzutreiben, um das dritte vordefinierte Volumen V3 des Medikaments mit der dritten Rate R3 zu verabreichen.

12. Medikamenten-Verabreichungsvorrichtung nach einem der Ansprüche 1 bis 10, wobei die Steuerung (8, 108) ferner dazu ausgestaltet ist, den Antrieb (104) mit variabler Geschwindigkeit anzutreiben, um ein Tupel vordefinierten Volumens Vn des Medikaments aus dem Behälter (4) mit einer Tupel-Rate Rn zu verabreichen, und wobei die Steuerung (8, 108) dazu ausgestaltet ist:
a. einen Endpunkt für eine Phase Pn-1 vor einer Tupel-Phase Pn zu identifizieren und
b. den Antrieb (104) mit variabler Geschwindigkeit während der Tupel-Phase Pn mit einer Tupel-Geschwindigkeit anzutreiben, um das Tupel vordefinierten Volumens Vn des Medikaments mit der Tupel-Rate Rn zu verabreichen.

13. Medikamenten-Verabreichungsvorrichtung nach einem der vorhergehenden Ansprüche, wobei die zweite Rate R2 kleiner als die erste Rate R1 ist.

## Revendications

1. Dispositif d'administration de médicaments comprenant :
un logement (2) configuré pour recevoir un récipient (4) contenant un volume de médicament ;
un actionneur (8) configuré pour initier une procédure d'administration de médicament ; et
un système d'entraînement (10) comprenant :
un composant d'entraînement axial (102, 102a, 102b) configuré pour entraîner l'avancement d'un bouchon (4a) fermant le récipient (4) ;
un entraînement à débit variable (104) configuré pour entraîner l'avancement du composant d'entraînement axial (102, 102a, 102b), l'entraînement à débit variable (104) comprenant un moteur (104a) configuré pour entraîner le composant d'entraînement axial ; et
un dispositif de commande (8 108) comprenant un processeur (112),
**caractérisé en ce que** le dispositif de commande (8, 108) est configuré pour :
a. entraîner l'entraînement à débit variable (104) à une première vitesse pendant une première phase P1 pour administrer un premier volume V1 prédéfini du médicament provenant du récipient (4) à un premier débit R1 ;
b. identifier un point final pour la première phase P1 en identifiant l'achèvement d'un nombre prédéterminé de rotations du moteur (104a) ; et
c. entraîner l'entraînement à débit variable (104) à une deuxième vitesse pendant une deuxième phase P2 pour administrer un deuxième volume prédéfini V2 du médicament à partir du récipient (4) à un deuxième débit R2.

2. Dispositif d'administration de médicament selon la revendication 1, le dispositif de commande (8, 108) étant en outre configuré pour identifier un point de début pour l'administration d'un médicament à partir du récipient (4), le point de début étant identifié en identifiant l'achèvement d'un événement préparatoire prédéfini, et la première phase P1 commençant au point de début.

3. Dispositif d'administration de médicament selon la revendication 1 ou la revendication 2, le composant d'entraînement axial (102, 102a, 102b) faisant partie d'un ensemble de vis télescopique (102) du système d'entraînement.

4. Dispositif d'administration de médicament selon l'une quelconque des revendications précédentes, le moteur (104a) étant un moteur (104a) entraîné par modulation de largeur d'impulsion (PWM).

5. Dispositif d'administration de médicament selon l'une quelconque des revendications 2 à 4, l'événement préparatoire prédéterminé étant l'un parmi :
l'achèvement d'une distance prédéterminée parcourue par le composant d'entraînement axial (102, 102a, 102b) ;
l'achèvement d'un nombre préparatoire prédéterminé de rotations du moteur (104a) ; et
l'achèvement d'une période de temps prédéterminée.

6. Dispositif d'administration de médicament selon l'une quelconque des revendications précédentes, le système d'entraînement (10) comprenant un codeur optique (410) pour surveiller une vitesse du moteur (104a) et fournir une rétroaction au dispositif de commande (8, 108).

7. Dispositif d'administration de médicament selon la revendication 6, le dispositif de commande (8, 108) étant configuré pour régler la puissance fournie au moteur (104a) en réponse à la vitesse surveillée du moteur (104a).

8. Dispositif d'administration de médicament selon l'une quelconque des revendications précédentes, le dispositif de commande (8, 108) étant configuré pour commander l'entraînement à débit variable (104) afin qu'il fonctionne à une vitesse préparatoire pendant une phase préparatoire P0 jusqu'à l'achèvement de l'événement de préparation prédéterminé.

9. Dispositif d'administration de médicament selon la revendication 8, la vitesse préparatoire étant supérieure à la première vitesse S1 et à la deuxième vitesse S2.

10. Dispositif d'administration de médicament selon l'une quelconque des revendications précédentes, une période d'administration de médicament (T_{D}) étant définie comme un temps pris pour achever la première phase (T_{P1}) et un temps pris pour achever la deuxième phase (T_{P2}), et la période d'administration de médicament (T_{D}) étant :
au moins 2,5 minutes ;
au moins 5 minutes ;
au moins 10 minutes ;
au moins 30 minutes ;
au moins 60 minutes ;
au moins 120 minutes ;
au moins 240 minutes ; ou
au moins 300 minutes.

11. Dispositif d'administration de médicament selon l'une quelconque des revendications 1 à 9, le dispositif de commande (8, 108) étant en outre configuré pour entraîner l'entraînement à débit variable (104) afin d'administrer un troisième volume prédéfini V3 du médicament à partir du récipient (4) à un troisième débit R3, et
le dispositif de commande (8, 108) est configuré pour :
a. identifier un point final pour la deuxième phase P2 ; et
b. entraîner l'entraînement à débit variable (104) à une troisième vitesse pendant une troisième phase P3, pour administrer le troisième volume prédéfini V3 du médicament au troisième débit R3.

12. Dispositif d'administration de médicament selon l'une quelconque des revendications 1 à 10, le dispositif de commande (8, 108) étant configuré pour entraîner l'entraînement à débit variable (104) afin d'administrer un volume prédéfini de tuple Vn du médicament à partir du récipient (4) à un débit de tuple Rn, et
le dispositif de commande (8, 108) est configuré pour :
a. identifier un point final pour une phase Pn-1 avant une phase Pn ; et
b. entraîner l'entraînement à débit variable (104) à une vitesse de tuple pendant la phase de tuple Pn pour administrer le volume prédéfini de tuple Vn du médicament au débit de tuple Rn.

13. Dispositif d'administration de médicament selon l'une quelconque des revendications précédentes, le deuxième débit R2 étant inférieure au premier débit R1.
